# EUROPEAN PATENT SPECIFICATION

(11) **EP 4 539 692 B1**
(45) Date of publication and mention of the grant of the patent: **08.04.2026**
(21) Application number: 23738065.4
(22) Date of filing: 06.06.2023
(51) Int. Cl.: A41B 1/08, A41D 1/04, A41D 27/24, A61M 21/02

(54) **A GARMENT**
KLEIDUNGSSTÜCK
VÊTEMENT

(30) Priority: 14.06.2022 GB 202208750
(43) Date of publication of application: 23.04.2025
(73) Proprietor: PALFREY, Matt, DE1 2RJ (GB)
(72) Inventor: PALFREY, Max, Derby DE1 2RJ (GB); PALFREY, Matt, Derby DE1 2RJ (GB)
(74) Representative: Potter Clarkson
(86) International application number: PCT/GB2023/000033
(87) International publication number: WO 2023/242529

(56) References cited:
- US-A1- 2021 308 414
- US-A1- 2022 151 310

## Description

### Field of the Invention

The present invention relates to a garment, such as a T-shirt, that is arranged to provide calming stimulation to a wearer.

### Background

Many people have been recognised to have different emotional, psychological, sensory needs, for example finding that they need stimulation when emotionally or nervously active.

Such stimulation may involve activating a plurality of nerve signals for sensory information such as texture, temperature, and other tactile sensations, which can soothe neurodiverse and neurotypical persons.

A person with tactile sensory needs has a tendency to take repetitive actions which may be noticeable and unusual. This can be embarrassing for the person.

### Prior Art

US 2021 308 414 A1 (FOSTER) discloses clothing outfitted with tactile stimulation packages, the packages preferably filled with 1 cm flat round marbles, 2 cm plastic prism gems, ½ cm plastic octagonal beads, 1 cm round plastic spheres/marbles, 2 cm glass marbles, memory foam, and gel.

US 2021 0 289 864 (OBLETZ) article of clothing comprising a pocket coupled to its body structure; a sensory object coupled to the interior of the pocket, namely a fidget spinner, fidget cube, stretchy string fidget, finger fling, squishy toy, a pad with a plurality of arms, coupled rings, flippy bike chain fidget or marble within a tube.

US 2015 0 273 178 (JOHNSON) discloses clothing system specifically designed with integrated tactile sensory comfort items integrated with the articles of clothing, or person specific textured patches which may be placed in pockets, as well as inside and outside of shirts, shorts, pants, and sweatshirts and other articles of clothing.

The present invention arose in order to overcome problems suffered by existing devices.

### Summary of the Invention

According to the present invention there is provided a garment having a seam including an interaction device comprising a base and at least one interactive button, which button is operable to move from a first position extending from a first face of the base to a second position extending from an opposed second face of the base.

The garment according to the invention is designed to provide sensory feedback to the wearer, by way of the interaction device included in the garment's seam, wherein said device is operable to enhance the user's tactile perception, proprioception, or overall sensory experience.

It may be envisaged that the garment comprises a T-shirt or torso-covering garment, but it may be envisaged that embodiments of the invention may include other garments such as hats, shorts or socks.

In some embodiments at least one part of the base is secured in or to the garment, whereby the device is operable without displacement from, or substantially even on, the garment, such that the movements are not obtrusive.

In this way the interactive button may thereby provide a self-contained and unobtrusive means of obtaining movement via a haptic stimulation object which enables stress relief and relaxation to users who suffer from a stress or anxiety disorder, for example users on the autistic spectrum.

The device may allow the user to move the button repeatedly from one position to another, without providing obtrusive physical movements which the user may be likely to feel self-conscious about.

In some embodiments the device is securely held within the garment using non-intrusive stitches. This ensures the device's stable position, preventing any undesired movement or displacement during normal usage and thereby eliminating potential sensory irritation to the wearer.

In some embodiments the device is held within the garment by a double turned hem, wherein the fabric of the garment is folded over the device so as to securely encase the device within the hem. The double turned hem creates a smooth, flat surface, allowing stitching to hold the device in place, without any raised seams, thereby ensuring a seamless and uninterrupted tactile experience for the wearer and eliminating any potential discomfort or irritation caused by raised or protruding elements.

In some embodiments seams throughout the garment are formed only by non-intrusive stitches. The non-intrusive stitches prioritise the wearer's comfort and minimise sensory irritation. In particular, the seams of the garment are carefully formed using stitches that maintain a low profile and prevent any raised or bulky seams that could cause discomfort. These non-intrusive stitches ensure a smooth and seamless surface, both inside and outside the garment, thus enhancing the overall wearing experience and preventing any potential sensory irritation for the wearer.

In some embodiments stitches throughout the garment utilise flat felled seams to further minimize sensory irritation. The flat felled seams involve folding the fabric of the garment over itself and stitching it down, creating a strong and smooth seam with no raw edges exposed to the wearer's skin. By employing flat felled seams, the garment ensures an irritation-free experience for the wearer. The flat nature of the seams prevents any rough or abrasive edges that could potentially cause discomfort or sensory irritation, thus enhancing the overall comfort and usability of the garment.

In some embodiments the device comprises a plurality of buttons, such that the user may be enabled to move one button in one direction and a second button in a reverse direction, providing a variety of movements within a minimal range of visible movements and space.

Additionally or alternatively the presence of the devices may provide reassurance to the user.

In some embodiments the device is located along the bottom edge of the garment in use. In this way the devices are liable to be located close to the user's hands at a majority of times. In other embodiments the device or devices may be located at other seams, for example at existing or standard hems, and/or included in a dedicated seam, for example included in a breast location such as a pocket.

In some embodiments the garment is made of soft fabric. The soft fabric provides a gentle and comfortable feel against the wearer's skin. The soft fabric used in the garment enhances the tactile experience for the wearer, ensuring a pleasant sensation and reducing the likelihood of any potential discomfort or irritation. The softness of the fabric contributes to the overall comfort and usability of the garment, allowing the wearer to fully appreciate the sensory feedback provided by the enclosed device without any distractions or unpleasant sensations caused by rough or harsh materials.

In some embodiments the garment comprises a soft pilled or brushed finish fabric. In this way the garment may provide a reassuring, comforting feel to a wearer. The garment may be envisaged to be a T-shirt, for example a rounded neck T-shirt with short sleeves, so as to provide an easy to wear, standard issue garment that is not liable to concern the user. Other embodiments may comprise sweaters, hooded jumpers, or other long- or short-sleeved format known in the art.

In some embodiments the garment comprises bamboo. This may be sustainable, comfortable, compostable, natural and breathable. In some embodiments the garment is comprised of Modal Knit. This is 94% micromodal and 6% Elastane, whereby micromodal a natural fibre made from the pulp of beech trees.

In some embodiments the garment is devoid of any interior labels or tags. Such a garment is intentionally designed without any interior labels or tags to enhance the wearer's comfort and prevent potential sensory irritation. By eliminating interior labels or tags, the garment eliminates the presence of labels or tags that can cause itching, scratching, or discomfort against the wearer's skin. This omission ensures a seamless and uninterrupted tactile experience, allowing the wearer to fully enjoy the sensory feedback provided by the enclosed device without any distractions or irritations caused by interior labels or tags.

In some embodiments the device is located to a rear area of the garment in use. In this way the user may be enabled to reach behind them to access the devices quickly and without difficulty. For example the device or devices may be located proximate rear trouser pockets so as to be readily accessible at all times.

In some embodiments the device is located to a fore area of the garment in use. In this way the user may be enabled to reach behind them to access the devices quickly and without difficulty. For example the device or devices may be located proximate front trouser pockets so as to be readily accessible at all times.

Some embodiments may be envisaged to have devices in the seam to fore and rear.

In some embodiments the device is located in a pocket. For example the seam may be to an outer face or an inner face of the garment and may have an open top edge.

In some embodiments the garment may have covered hems to an inner face of the garment, so as to limit skin contact of the user with sewn seams, and the discomfort this can cause certain people.

In some embodiments the device is located under a displaceable hinged flap. This flap may allow the device to be displaced wholly or partially from the garment, and/or may hide the presence of the device and/or may limit skin contact with the user of hems or the device.

In some embodiments the device is sewn into the garment, or otherwise secured thereto, for example using adhesive.

In some embodiments the device is secured to the garment along one edge of the device only, so as to allow said side to act as a hinge for example, enabling the device to be hinged one way for use, and an opposed way for use.

In some embodiments a plurality of devices are spaced regularly along the seam, so as to provide a plurality of locations for the user's relief as appropriate to need and visibility or obtrusiveness.

In some embodiments sleeve cuffs of the garment are in the form of turn-back cuffs, and wherein overlock stitching is concealed inside the turn-back cuffs when folded back. This prevents direct contact of the stitching against the wearer's skin. The turned back cuffs of the garment are intentionally crafted to provide an additional layer of comfort and protection. When folded back, the cuff conceals the overlock stitching, ensuring that no rough or exposed edges come into contact with the wearer's skin. This design feature eliminates the potential for irritation or discomfort caused by the stitching, allowing the wearer to experience a smooth and irritation-free sensation while benefiting from the sensory feedback provided by the enclosed device.

A preferred embodiment of the invention will now be described by way of example only and with reference to the Figures in which:

### Brief Description of Figures

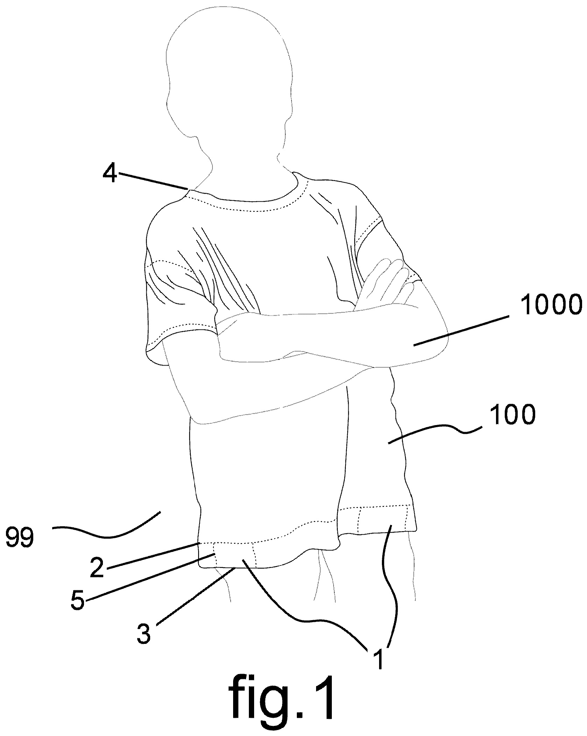

Figure 1 shows an isometric view of an embodiment of the device according to the present invention, viewed from in front, being worn by a user;

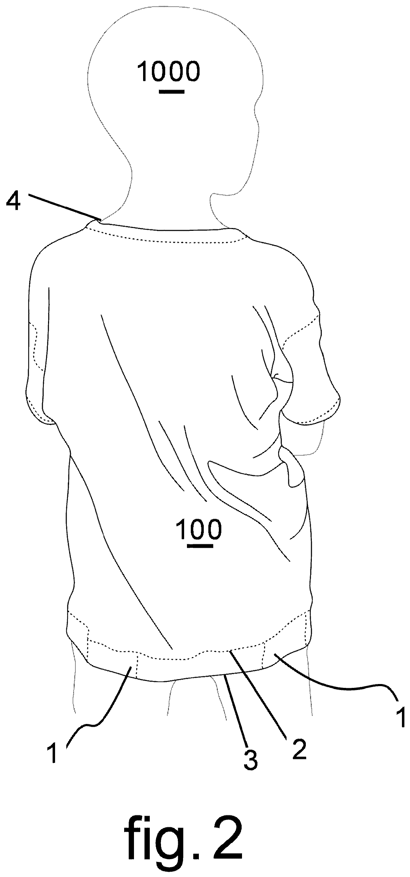

Figure 2 shows an isometric view of the embodiment of the device as shown in Figure 1, viewed from behind;

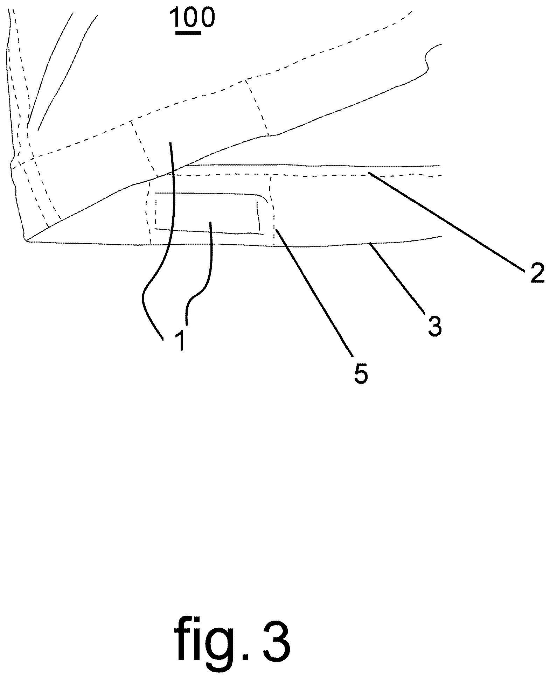

Figure 3 shows a detail isometric view of the embodiment of the device shown in Figure 1;

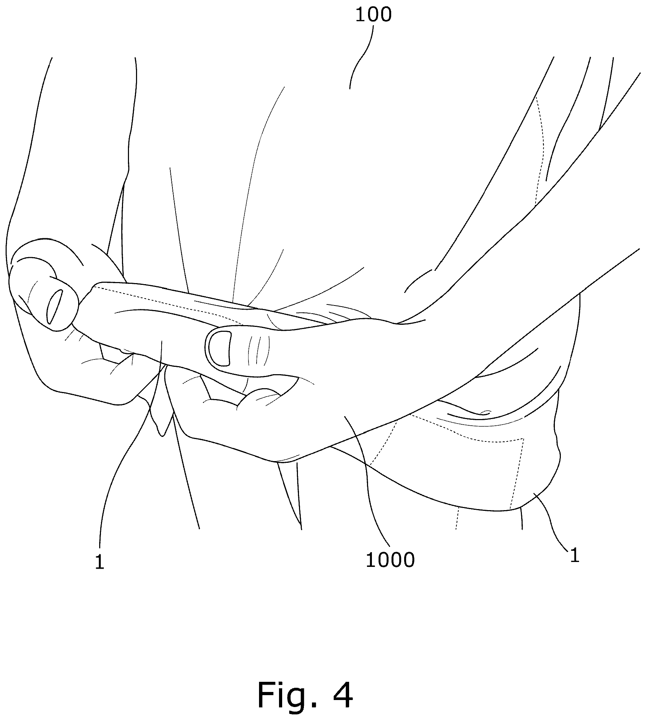

Figure 4 shows a sketch of a detail isometric view of the embodiment of the device shown in Figure 1, in use;
   And
Figure 5 shows a sketch of a plan view of a device for use in the embodiment of the device shown in Figure 1.

### Detailed Description of Figures

With reference to the figures there is shown an embodiment of a torso covering garment 99 having a hemmed seam 3, the seam 3 including an interaction device 1 comprising a base and at least one interactive button, which button is adapted to move from a first position extending from a first face of the base to a second position extending from an opposed second face of the base.

The device in the pictured embodiment comprises two buttons 11 on a base 12. The buttons are thin flexible inelastic material which pushes through a relatively more rigid base from one side of the base to the other.

Further embodiments may include more buttons, and/or a multipartite and/or articulated base, so as to allow twisting of the base and/or rearrangement of buttons.

More particularly the garment is a T-shirt 100 with short sleeves. The T-shirt has a round neck 4 and is made of a super-soft bamboo fabric, for complete comfort.

The garment has a wide hem 3 at its bottom edge.

The hem has a top sewn seam 2 and vertical separation seams 5.

The separation seams are arranged to delineate the location of the device 1 which is included between the front and rear of the seam material.

The pictured embodiment of the garment includes four devices 1, two of which are located notionally above the front pockets of a pair of trousers to fore of the garment, and so as to provide easy and unobtrusive access to the devices in use. In this way a user 1000 will be less concerned about being noticed when using the devices.

The only visible sign of the presence of the devices will be the vertical separators 5. Such separators may additionally or alternatively provide a convenient haptic feedback to the user 1000, either to identify the location of the devices and/or reassure the user 1000 of the presence of the device.

Additionally or alternatively the devices may be placed into a trouser pocket in use, where the garment is long enough, so as to be even less obtrusive. Two devices 1 are included in the rear of the garment, being located notionally approximately above rear pockets on trousers in use.

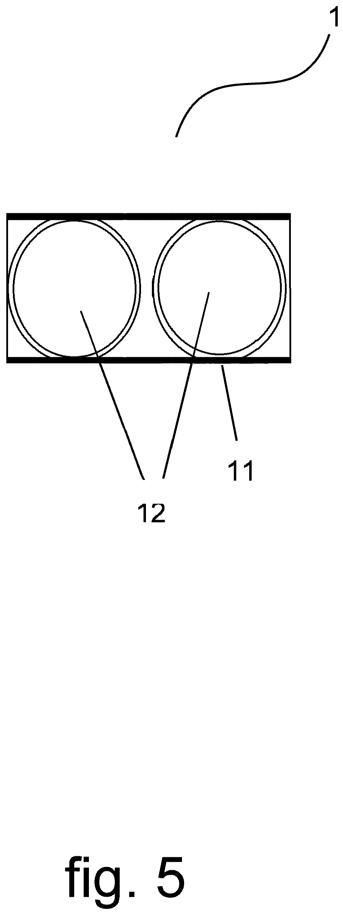

As shown in Figure 5 the envisaged devices each comprise two buttons on a base. The user 1000 may thereby be enabled to push the buttons from one side of the base to the opposed side and vice-versa so as to alleviate stress or otherwise occupy the hands.

The invention has been described by way of examples only and it will be appreciated that variation may be made to the above-mentioned embodiments without departing from the scope of protection as defined by the claims.

## Claims

1. A garment (99,100) having a seam (3) including an interaction device (1), **characterised by** the interaction device (1) comprising a base (12) and at least one interactive button (11), which button (11) is operable to move from a first position extending from a first face of the base (12) to a second position extending from an opposed second face of the base (12).

2. A garment (99,100) according to claim 1 wherein at least one part of the base (12) is secured in or to the garment (99,100), whereby the device (1) is operable without displacement from, or even on, the garment (99,100).

3. A garment (99,100) according to any preceding claim, wherein said device (1) is securely held within the garment (99,100) using non-intrusive stitches.

4. A garment (99,100) according to any preceding claim wherein the device (1) is held within the garment (99,100) by a double turned hem, wherein the fabric of the garment (99,100) is folded over the device (1) so as to securely encase the device (1) within the hem.

5. A garment (99,100) according to any preceding claim, wherein seams (3) throughout the garment (99,100) are formed only by non-intrusive stitches, and/or wherein stitches throughout the garment (99,100) utilise flat felled seams.

6. A garment (99,100) according to any preceding claim, wherein the device (1) is located along the bottom edge of the garment (99,100).

7. A garment (99,100) according to any preceding claim, wherein the garment (99,100) is made of soft fabric or soft pilled fabric.

8. A garment (99,100) according to any preceding claim wherein the garment (99,100) is formed in bamboo or Modal Knit.

9. A garment (99,100) according to any preceding claim, wherein the garment (99,100) is devoid of any interior labels or tags.

10. A garment (99,100) according to any preceding claim wherein the device (1) is located to fore or rear of the garment (99,100) in use.

11. A garment (99,100) according to any preceding claim wherein the device (1) is located in a pocket or under a displaceable hinged flap.

12. A garment (99,100) according to any preceding claim wherein the device (1) is secured to the garment (99,100) along one edge of the device (1) so as to allow hinging.

13. A garment (99,100) according to any preceding claim comprising a plurality of devices (1) spaced regularly along the seam (3).

14. A garment (99,100) according to any preceding claim, wherein sleeve cuffs of the garment (99,100) are in the form of turn-back cuffs, and wherein overlock stitching is concealed inside the turn-back cuffs when folded back.

15. A torso covering garment (99,100) according to any of the preceding claims.

## Patentansprüche

1. Kleidungsstück (99, 100), das eine Naht (3) aufweist, die eine Interaktionsvorrichtung (1) einschließt, **dadurch gekennzeichnet, dass** die Interaktionsvorrichtung (1) eine Basis (12) und mindestens einen interaktiven Knopf (11) umfasst, wobei der Knopf (11) betreibbar ist, um sich von einer ersten Position, die sich von einer ersten Fläche der Basis (12) erstreckt, in eine zweite Position zu bewegen, die sich von einer gegenüberliegenden zweiten Fläche der Basis (12) erstreckt.

2. Kleidungsstück (99, 100) nach Anspruch 1, wobei mindestens ein Teil der Basis (12) in oder an dem Kleidungsstück (99, 100) befestigt ist, wodurch die Vorrichtung (1) ohne Verschiebung von dem oder sogar auf dem Kleidungsstück (99, 100) betätigt werden kann.

3. Kleidungsstück (99, 100) nach einem der vorstehenden Ansprüche, wobei die Vorrichtung (1) mithilfe von nichtintrusiven Nähten sicher innerhalb des Kleidungsstücks (99, 100) gehalten wird.

4. Kleidungsstück (99, 100) nach einem der vorstehenden Ansprüche, wobei die Vorrichtung (1) durch einen doppelt eingeschlagenen Saum innerhalb des Kleidungsstücks (99, 100) gehalten wird, wobei der Stoff des Kleidungsstücks (99, 100) so über die Vorrichtung (1) gefaltet ist, dass er die Vorrichtung (1) sicher innerhalb des Saums umschließt.

5. Kleidungsstück (99, 100) nach einem der vorstehenden Ansprüche, wobei Nähte (3) im gesamten Kleidungsstück (99, 100) ausschließlich durch nicht-intrusive Stiche gebildet werden und/oder wobei Stiche im gesamten Kleidungsstück (99, 100) flache Kappnähte verwenden.

6. Kleidungsstück (99, 100) nach einem der vorstehenden Ansprüche, wobei die Vorrichtung (1) entlang der Unterkante des Kleidungsstücks (99, 100) angeordnet ist.

7. Kleidungsstück (99, 100) nach einem der vorstehenden Ansprüche, wobei das Kleidungsstück (99, 100) aus weichem Stoff oder weichem gewellten Stoff hergestellt ist.

8. Kleidungsstück (99, 100) nach einem der vorstehenden Ansprüche, wobei das Kleidungsstück (99, 100) aus Bambus oder Modalstrick gebildet ist.

9. Kleidungsstück (99, 100) nach einem der vorstehenden Ansprüche, wobei das Kleidungsstück (99, 100) keine inneren Etiketten oder Anhänger aufweist.

10. Kleidungsstück (99, 100) nach einem der vorstehenden Ansprüche, wobei die Vorrichtung (1) bei Gebrauch an der Vorderseite oder Rückseite des Kleidungsstücks (99, 100) angeordnet ist.

11. Kleidungsstück (99, 100) nach einem der vorstehenden Ansprüche, wobei die Vorrichtung (1) in einer Tasche oder unter einer verschiebbaren aufklappbaren Klappe angeordnet ist.

12. Kleidungsstück (99, 100) nach einem der vorstehenden Ansprüche, wobei die Vorrichtung (1) entlang einer Kante der Vorrichtung (1) an dem Kleidungsstück (99, 100) befestigt ist, um ein Schwenken zu ermöglichen.

13. Kleidungsstück (99, 100) nach einem der vorstehenden Ansprüche, umfassend eine Vielzahl von Vorrichtungen (1), die regelmäßig entlang der Naht (3) beabstandet sind.

14. Kleidungsstück (99, 100) nach einem der vorstehenden Ansprüche, wobei Ärmelmanschetten des Kleidungsstücks (99, 100) in Form von Umschlagmanschetten vorliegen und wobei Overlocknähte innerhalb der Umschlagmanschetten verdeckt sind, wenn diese umgeschlagen sind.

15. Oberkörperbekleidungsstück (99, 100) nach einem der vorstehenden Ansprüche.

## Revendications

1. Vêtement (99,100) ayant une couture (3) comportant un dispositif d'interaction (1), **caractérisé en ce que** le dispositif d'interaction (1) comprend une base (12) et au moins un bouton interactif (11), lequel bouton (11) peut être actionné pour se déplacer d'une première position s'étendant à partir d'une première face de la base (12) jusqu'à une seconde position s'étendant à partir d'une seconde face opposée de la base (12).

2. Vêtement (99,100) selon la revendication 1 dans lequel au moins une partie de la base (12) est fixée dans le vêtement (99,100) ou sur celui-ci, moyennant quoi le dispositif (1) peut être actionné sans être déplacé du vêtement (99,100) ou même sur celui-ci.

3. Vêtement (99,100) selon l'une quelconque revendication précédente, dans lequel ledit dispositif (1) est solidement maintenu à l'intérieur du vêtement (99,100) à l'aide de points de couture non intrusifs.

4. Vêtement (99,100) selon l'une quelconque revendication précédente dans lequel le dispositif (1) est maintenu à l'intérieur du vêtement (99,100) par un ourlet double rentré, dans lequel le tissu du vêtement (99,100) est plié sur le dispositif (1) de manière à enfermer solidement le dispositif (1) à l'intérieur de l'ourlet.

5. Vêtement (99,100) selon l'une quelconque revendication précédente, dans lequel les coutures (3) sur tout le vêtement (99,100) sont formées uniquement par des points de couture non intrusifs, et/ou dans lequel les points de couture sur tout le vêtement (99,100) utilisent des coutures rabattues.

6. Vêtement (99,100) selon l'une quelconque revendication précédente, dans lequel le dispositif (1) est situé le long du bord inférieur du vêtement (99,100).

7. Vêtement (99,100) selon l'une quelconque revendication précédente, dans lequel le vêtement (99,100) est fait d'un tissu doux ou d'un tissu à poils doux.

8. Vêtement (99,100) selon l'une quelconque revendication précédente dans lequel le vêtement (99,100) est composé de bambou ou de maille de modal.

9. Vêtement (99,100) selon l'une quelconque revendication précédente, dans lequel le vêtement (99,100) est dépourvu de toute étiquette ou marque intérieure.

10. Vêtement (99,100) selon l'une quelconque revendication précédente dans lequel le dispositif (1) est situé à l'avant ou à l'arrière du vêtement (99,100) lors de son utilisation.

11. Vêtement (99,100) selon l'une quelconque revendication précédente dans lequel le dispositif (1) est situé dans une poche ou sous un rabat articulé déplaçable.

12. Vêtement (99,100) selon l'une quelconque revendication précédente dans lequel le dispositif (1) est fixé au vêtement (99,100) le long d'un bord du dispositif (1) de manière à permettre l'articulation.

13. Vêtement (99,100) selon l'une quelconque revendication précédente comprenant une pluralité de dispositifs (1) régulièrement espacés le long de la couture (3).

14. Vêtement (99,100) selon l'une quelconque revendication précédente, dans lequel les bas de manches du vêtement (99,100) sont sous la forme de poignets revers, et dans lequel les points de surjet sont dissimulées à l'intérieur des poignets revers lorsqu'ils sont repliés.

15. Vêtement couvrant le torse (99,100) selon l'une quelconque des revendications précédentes.
